# EUROPEAN PATENT SPECIFICATION

(11) **EP 2 229 172 B1**
(45) Date of publication and mention of the grant of the patent: **10.09.2014**
(21) Application number: 08857354.8
(22) Date of filing: 02.12.2008
(51) Int. Cl.: A61K 31/519, A61P 9/00, A61P 9/10

(54) **TICAGRELOR FOR TREATMENT OF ABDOMINAL AORTIC ANEURYSMS**
TICAGRELOR ZUR BEHANDLUNG VON BAUCHAORTENANEURYSMEN
TICAGRELOR POUR LE TRAITEMENT D'ANÉVRYSMES AORTIQUES ABDOMINAUX

(30) Priority: 03.12.2007 EP 07301616
(43) Date of publication of application: 22.09.2010
(73) Proprietor: AstraZeneca AB, 151 85 Södertälje (SE)
(72) Inventor: MICHEL, Jean-Baptiste, F-75018 Paris (FR)
(74) Representative: Berry, Ian Gordon
(86) International application number: PCT/SE2008/051386
(87) International publication number: WO 2009/072967

(56) References cited:
- EP-A1- 1 493 745
- WO-A1-00/34283
- WO-A1-01/92262
- WO-A1-97/03084
- WO-A1-99/05143
- WO-A1-02/096428
- WO-A2-2009/007675
- US-A1- 2007 265 282
- R.T. OWEN ET AL: "AZD6140", DRUGS OF THE FUTURE, vol. 32, no. 10, 1 January 2007 (2007-01-01), page 845, XP055024216, ISSN: 0377-8282, DOI: 10.1358/dof.2007.032.10.1133832
- DATABASE MEDLINE [Online] US NATIONAL LIBRARY OF MEDICINE (NLM), BETHESDA, MD, US; June 1992 (1992-06), ETANI H ET AL: "[Platelet accumulation in abdominal aortic aneurysms and the effect of antiplatelet drugs: assessment by indium platelet scintigraphy].", XP002687010, Database accession no. NLM1640649 & KAKU IGAKU. THE JAPANESE JOURNAL OF NUCLEAR MEDICINE JUN 1992, vol. 29, no. 6, June 1992 (1992-06), pages 651-657, ISSN: 0022-7854
- LLOYD G M ET AL: "Patients with abdominal aortic aneurysm: Are we missing the opportunity for cardiovascular risk reduction?", JOURNAL OF VASCULAR SURGERY, C.V. MOSBY CO., ST. LOUIS, MO, US, vol. 40, no. 4, 1 October 2004 (2004-10-01), pages 691-697, XP004584815, ISSN: 0741-5214, DOI: 10.1016/J.JVS.2004.06.039
- DIEHM N ET AL: "Current evidence and prospects for medical treatment of abdominal aortic aneurysms", VASA, vol. 34, no. 4, November 2005 (2005-11), pages 217-223, XP9164626, ISSN: 0301-1526

## Description

### Field of the invention

The present invention is directed to the use of a P2Y₁₂ receptor (also known as P*_{2T}*, P2Y_{ADP} or P2T_{AC}) antagonist in the treatment of abdominal aortic aneurysms.

### Background of the invention

Abdominal aortic aneurysm (AAA) is a frequent form of atherothrombotic disease which is highly age- and gender-related (Bengtsson H at el. (1996), Ann N Y Acad Sci 800:1-24). AAA pathophysiology involves extracellular matrix proteolysis (Michel JB (2001), Arterioscler Thromb Vasc Biol 21:1389-1392), smooth muscle cell (SMC) disappearance (Michel JB (2003), Arterioscler Thromb Vasc Biol 23:2146-2154), inflammatory cell infiltration, and an absence of cell colonization and healing (Fontaine V et al. (2004), Am J Pathol 164:2077-2087). It was recently shown that the dynamic biology of the mural thrombus, including luminal fibrinogenesis (Touat Z et al. (2006), Am J Pathol 168:1022-1030) and abluminal fibrinolysis (Carrell TW et al. (2006), Vascular 14:9-16; Fontaine V et al. (2002), Am J Pathol 161:1701-1710), plays a significant role in AAA progression. In this context, the interaction between activated platelet aggregates and polymorphonuclear leukocyte (PMN) recruitment (Sarda-Mantel L et al. (2006), Arterioscler Thromb Vasc Biol 26:2153-2159; Touat et al., 2006) plays a major role in the absence of cellular healing (Fontaine V et al. (2004), Am J Pathol 164:2077-2087).

Studies showing that platelet accumulates in AAA and supporting the effect of antiplatelet drugs in the management of this condition are known (Etani H et al., The Japanese Journal of Nuclear medicine (1992) 29(4): 651-657). The use of antiplatelet agents in patients with AAA is reported in Lloyd GM et al. (2004), Journal of Vascular surgery 40(1): 691-697. Common therapies of AAA are reviewed for instance in Diehm N et al. (2005), Vasa-Journal of Vascular Diseases 34(4): 217-223.

It has been found that adenosine 5'-diphosphate (ADP) acts as a key mediator of thrombosis. ADP-induced platelet aggregation is mediated by the P2Y12 receptor subtype located on the platelet membrane. The P2Y12 receptor (also known as P*_{2T}*, P2Y_{ADP} or P2T_{AC})) is a G-protein coupled receptor primarily involved in mediating platelet activation/aggregation. The pharmacological characteristics of this receptor have been described, for example, in the references by Humphries et al. (1994), Br. J. Pharmacology, 113, 1057-1063, and Fagura et al. (1998), Br. J. Pharmacology, 124, 157-164. It has been shown that antagonists at this receptor offer significant improvements over other antithrombotic agents (see J. Med. Chem. (1999) 42, 213).

International Patent Application WO 99/05143 discloses generically a series of triazolo[4,5-*d*]pyrimidine compounds having activity as P*_{2T}* (also known as P2Y₁₂, P2Y_{ADP} or P2T_{AC}) antagonists. Recently, a new class of direct (non-prodrug) P*_{2T}* receptor antagonists has been described which offers significant improvements over other antithrombotic agents. International Patent Application WO 00/34283 discloses novel "direct" P_{2T} receptor antagonists, including compounds of formula (I). Crystalline and amorphous forms of a triazolo(4,5-d)pyrimidine compound is disclosed in WO01/92262.

The use of {1*S*-[1α,2α,3β(1*S**,2*R**),5β]}-3-(7-{[2-(3,4-difluorophenyl) cyclopropyl]amino}-5-(propylthio)-3*H*-1,2,3-triazolo[4,5-*d*]pyrimidin-3-yl)-5-(2-hydroxyethoxy)cyclopentane-1,2-diol as antiplatelet therapeutic agent, its action as P2Y₁₂(P_{2T}) antagonist and pre-clinical and clinical studies are reviewed in Owen RT et al. (2007), Drugs of the Future 32(10): 845-853. EP 1 493 745 A1 discloses the use of this compound as antiplatelet agent for use in the treatment of arterial thrombotic complication.

### Brief description of the figures

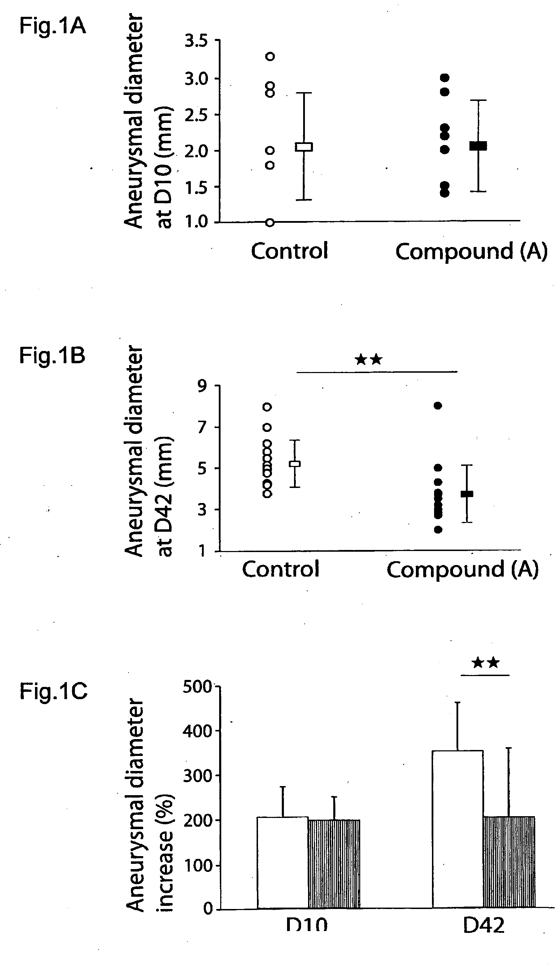

**Fig. 1****.** Prevention of aneurysmal expansion by Compound (A) treatment. Measurement of the external aneurysmal diameter after aneurysm induction at Day 10 (a) and Day 42 (b). The external diameter was similar in control (open square) and Compound (A)-treated (solid square) rats after 10 days (a), but was significantly lower in the Compound (A) group after 42 days of treatment (b). (c) The increase in aneurysmal diameter was similar for the two groups at Day 10 but was significantly reduced in the Compound (A)-treated rats (solid bars) at Day 42. Indeed, no increase in aneurysmal diameter was observed between Days 10 and 42 for Compound (A)-treated rats. **, *P* < 0.001.

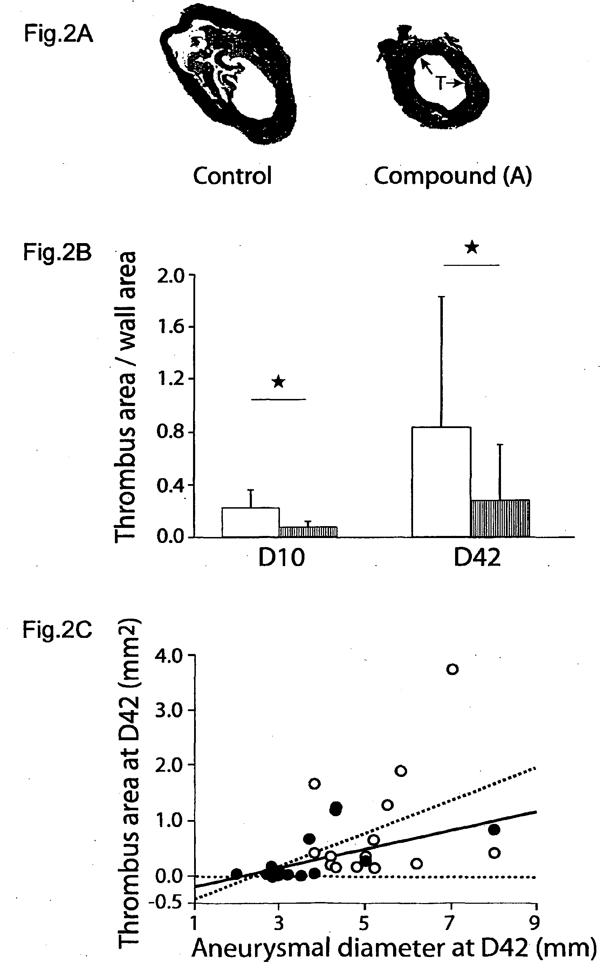

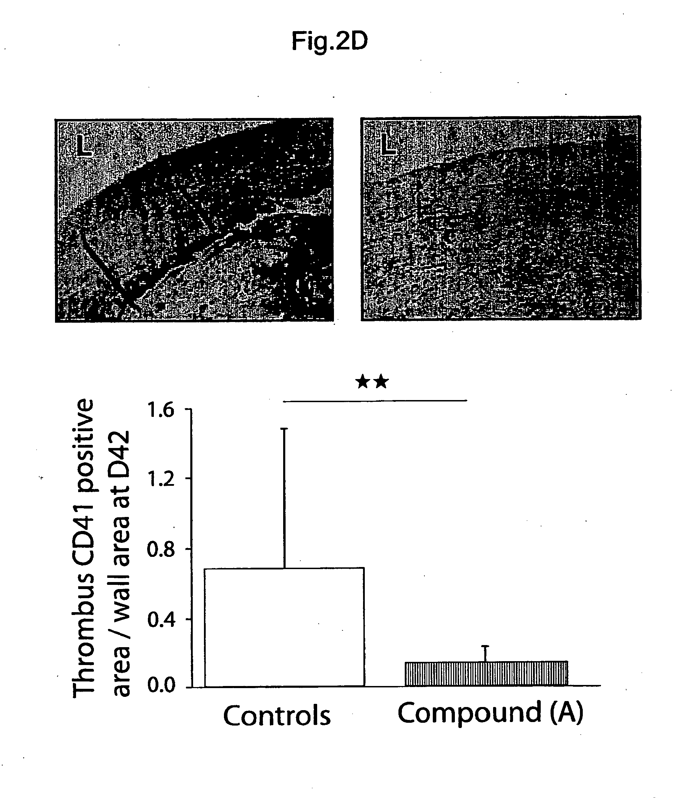

**Fig. 2****.** Inhibition of mural thrombus development by Compound (A) treatment. (a) Picro sirius red staining of representative aneurysms from control (left) and Compound (A)-treated rats (right) at Day 42. The aortic diameter and the thrombus (**T**) area were greater in aneurysms from control as compared to Compound (A)-treated rats. Note that the deposition of collagen (red staining) within the thrombus is only observed in the Compound (A) group. (b) The thrombus area/wall area increased between Days 10 and 42 in both groups of rats but was significantly reduced by Compound (A) treatment (solid bars) both at Days 10 and 42. (c) Intragroup analysis showed that the larger aneurysmal diameter at Day 42 was associated with a greater thrombus area (*R²* = 0.13 and *R²* = 0.40 for control (open circles) and Compound (A)-treated rats (solid circles), respectively, *P* < 0.05). (d) Immunostaining of CD41 (integrin a_{Iib}, a platelet marker) within the mural thrombus of aneurysms from control (left) and Compound (A)-treated rats (right) at Day 42. **L**: aortic lumen. Staining quantification shows that treatment for 42 days with Compound (A) significantly decreased the relative size of the CD41-positive area within the mural thrombus as compared to that seen in the control group. Original magnifications, x200. *,*P*< 0.05; **,*P*< 0.01.

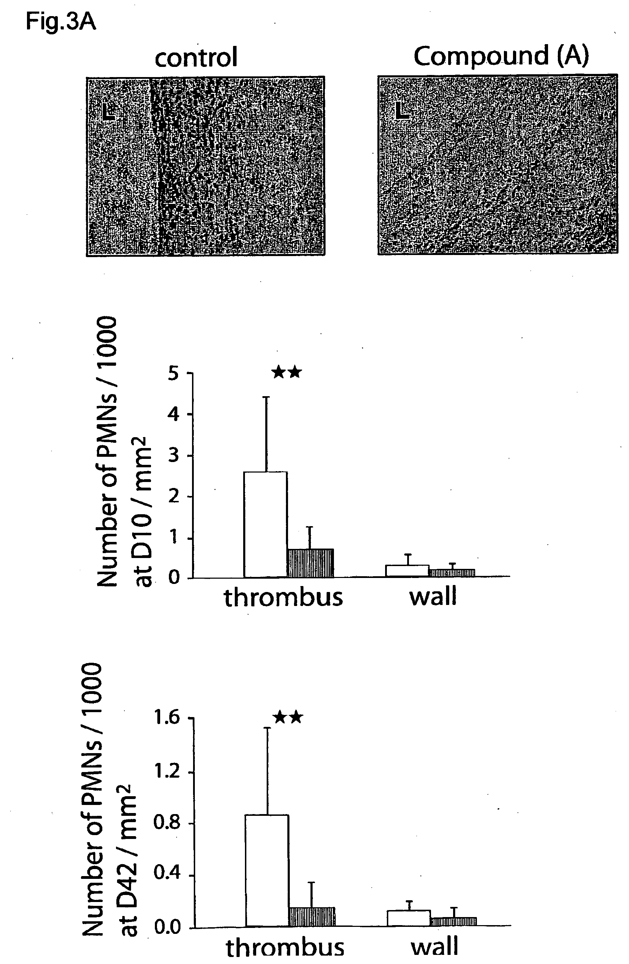

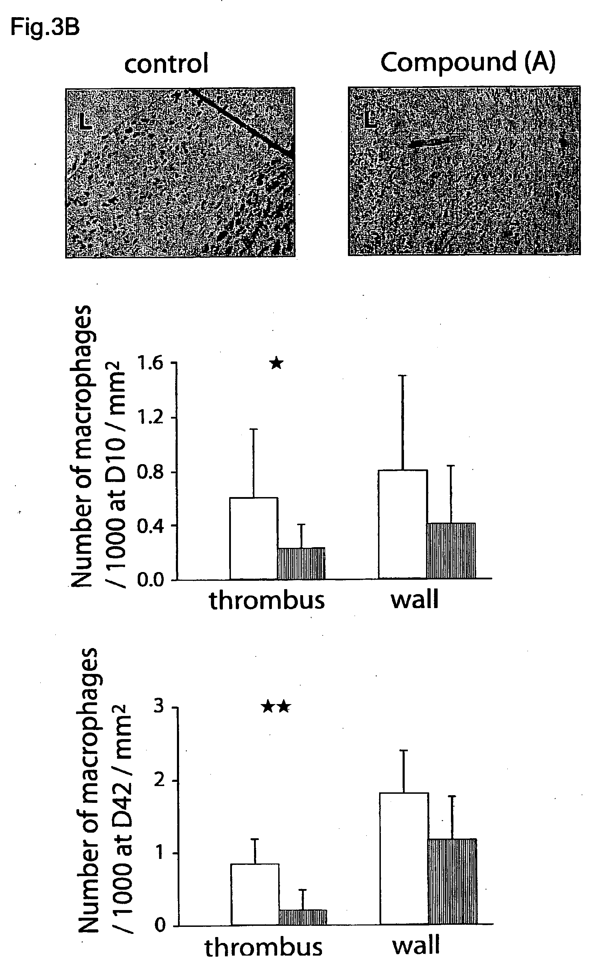

**Fig. 3****.** Inhibition of inflammatory infiltration by Compound (A) treatment. (a) Immunostaining of PMNs within the mural thrombus of aneurysms from control (left) and Compound (A)-treated rats (right) at Day 42. Quantification of PMN infiltration within the mural thrombus and the wall at Day 10 (middle panel) and 42 (lower panel). After 10 and 42 days, PMNs were mostly found within the mural thrombus of control aneurysms (open bars). Compound (A)-treatment (solid bars) inhibited the infiltration of PMNs into the thrombus at both times. (b) Immunostaining of macrophages within the mural thrombus of aneurysms from control (left) and Compound (A)-treated rats (right) at Day 42. Quantification of macrophage infiltration into the mural thrombus and the wall at Day 10 (middle panel) and 42 (lower panel). Macrophages were found within the mural thrombus as well as in the wall of control aneurysms (open bars). After 42 days, more macrophages were present within the wall than in the thrombus. Compound (A) treatment (solid bars) inhibited the infiltration of macrophages within the thrombus at both times. The decrease in macrophage content within the wall after 10 or 42 days of Compound (A) treatment did not reach statistical significance. **L**: aortic lumen. Original magnifications, x200. *, *P* < 0.05; **,*P*< 0.01.

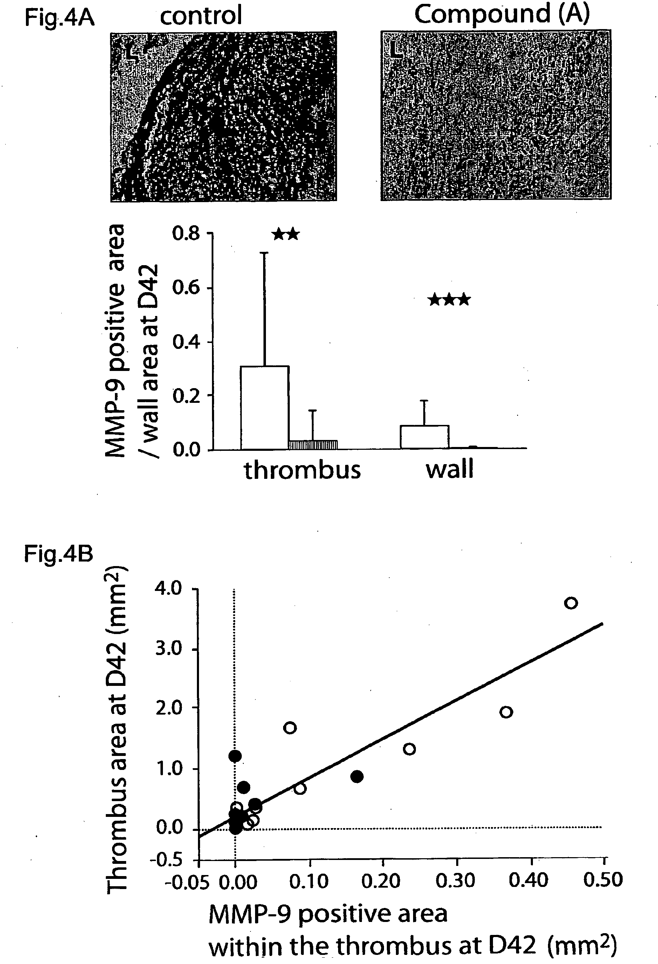

**Fig. 4****.** Decreased MMP-9 expression with Compound (A) treatment. (a) MMP-9 immunostaining within the mural thrombus of aneurysms from control (left) and Compound (A)-treated rats (right) at Day 42. Quantification of MMP-9 staining at Day 42 showed that the area occupied by MMP-9 staining relative to the wall area was greater within the thrombus than within the wall in control aneurysms (open bars). Compound (A) treatment strongly reduced the size of the MMP-9-positive area both within the thrombus and the wall (solid bars). **L**: aortic lumen. Original magnifications, x200. **, *P* < 0.001; ***, *P* < 0.0001. (b) There was a positive correlation between the size of the MMP-9-positive area within the thrombus and the total thrombus area at Day 42 when all rats were considered (controls, open circles; Compound (A)-treated, solid circles), *R²* = 0.79, *P* < 0.0001).

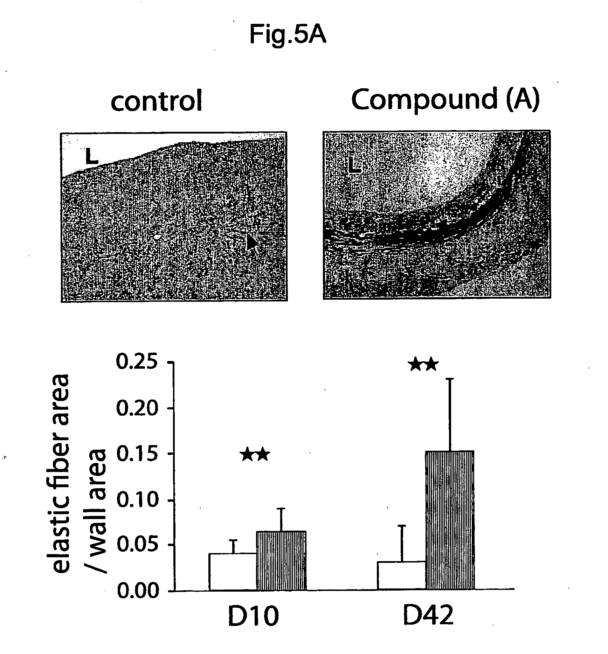

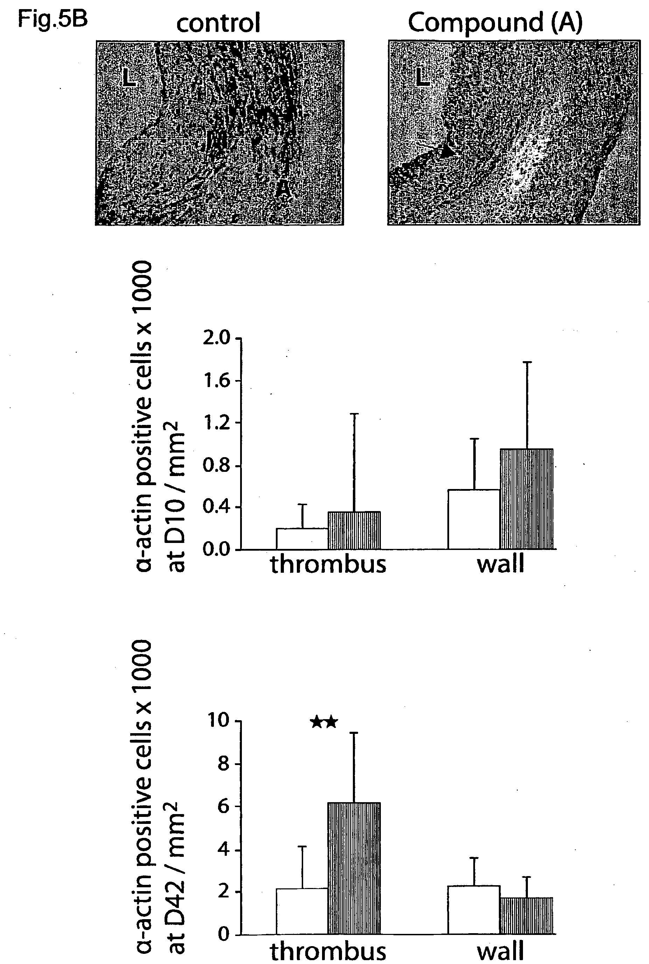

**Fig. 5****.** SMC colonization of the mural thrombus and preservation of elastic lamellae within the aneurysmal wall with Compound (A) treatment. (a) Orcein staining of aneurysms from control (left) and Compound (A)-treated rats (right) at Day 42. Arrows indicate the elastic lamellae. Quantification of the elastic lamellae area showed the beneficial effect of Compound (A) treatment (solid bars) at both Day 10 and 42. (b) Immunostaining of SMCs within the mural thrombus of aneurysms from control (left) and Compound (A)-treated rats (right) at Day 42. The arrow indicates SMCs within the thrombus. Density of SMCs within the mural thrombus and the wall at Day 10 (medium panel) and 42 (lower panel) increased between Days 10 and 42 in both groups. Compound (A) treatment (solid bars) improved the thrombus colonization by SMCs after 42 days compared to controls (open bars), whereas no effect of Compound (A) could be observed within the wall. **L**: lumen; **M**: media; **A**: adventitia. Original magnifications, x100. **, *P* < 0.001.

### Outline of the invention

The present invention is directed to the use of the compound {1*S*-[1α,2α,3β(1*S**,2*R**), 5β]}-3-(7-{[2-(3,4-difluorophenyl)cyclopropyl]amino}-5-(propylthio)-3*H*-1,2,3-triazolo [4,5-*d*]pyrimidin-3-yl)-5-(2-hydroxyethoxy)cyclopentane-1,2-diol for the manufacture of a medicament for the treatment of abdominal aortic aneurysms.

The present invention is directed to the compound {1*S*-[1α,2α,3β(1*S**,2*R**),5β]}-3-(7-{[2-(3,4-difluorophenyl)cyclopropyl]amino}-5-(propylthio)-3*H*-1,2,3-triazolo[4,5-*d*] pyrimidin-3-yl)-5-(2-hydroxyethoxy)cyclopentane-1,2-diol for use in the treatment of abdominal aortic aneurysms.

The use of a compound of formula (I): 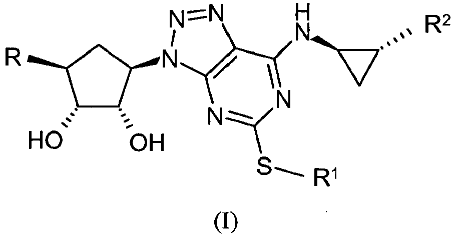 wherein:
R is CH₂OH or O(CH₂)₂OH;
R¹ is C₃₋₄ alkyl optionally substituted by three halogen atoms;
R² is phenyl or 3,4-difluorophenyl;
or a pharmaceutically acceptable derivative thereof,
for the treatment or prevention of abdominal aortic aneurysms is described herein.

Pharmaceutically acceptable derivatives of a compound of formula (I) include salts (e.g. pharmaceutically acceptable non-toxic organic or inorganic acid addition salts, such as a salt of hydrochloric, hydrobromic, nitric, sulphuric or acetic acid), solvates and solvates of salts.

A compound of formula (I) for the treatment or prevention of abdominal aortic aneurysms is described herein.

The use of a compound of formula (I) for the manufacture of a medicament for the treatment or prevention of abdominal aortic aneurysms is described herein.

The use of a compound of formula (I) in a method for the treatment or prevention of abdominal aortic aneurysms, wherein a pharmaceutically and pharmacologically effective amount of the compound of formula (I) is administered to a subject in need of such treatment or prevention is described herein.

The use of a compound of formula (I) in a method for the treatment or prevention of abdominal aortic aneurysms wherein the compound of formula (I) is administered to a subject in need of such treatment or prevention is described herein.

R¹ may be n-propyl, 3,3,3-trifluoropropyl or n-butyl. In one embodiment, R² may be 3,4-difluorophenyl. In one embodiment, the compound of formula (I) is compound (A):

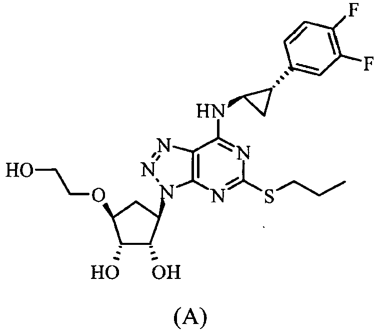

The compound (A) above is conventionally named {1*S*-[1α,2α,3β(1*S**,2*R**),5β]}-3-(7-{[2-(3,4-difluorophenyl)cyclopropyl]amino}-5-(propylthio)-3*H*-1,2,3-triazolo[4,5-*d*]pyrimidin-3-yl)-5-(2-hydroxyethoxy)cyclopentane-1,2-diol.

Suitable formulations for administering a compound of formula (I) are known in the art, and include those known from WO00/34283, WO2008/024044 and WO2008/024045. A pharmaceutical formulation of compound (I) may, and indeed will usually, contain various other ingredients known in the art, for example preservatives, stabilising agents, viscosity-regulating agents, emulsifying agents or buffering agents. Thus the pharmaceutical formulation of compound (I) will typically comprise a total amount of the compound of formula (I) in the range from 0.05 to 99 %w (per cent by weight), such as in the range from 0.10 to 70 %w, or in the range from 0.10 to 50 %w, all percentages by weight being based on total formulation.

Suitable doses of the compound of formula (I) can be determined by the medical practitioner or other skilled person, and will depend on the severity of the condition, and on the person to be treated, as well as the compound(s) which is/are employed. Suitable doses of active compound in the therapeutic and/or prophylactic treatment of mammalian, especially human, patients include those which give a mean plasma concentration of up to 10 µmol/L, for example in the range 0.001 to 10 µmol/L over the course of treatment of the relevant condition. In any event, the physician, or the skilled person, will be able to determine the actual dosage which will be most suitable for an individual person, which is likely to vary with the condition that is to be treated, as well as the age, weight, sex and response of the particular person to be treated. The above-mentioned dosages are exemplary of the average case. There can, of course, be individual instances where higher or lower dosage ranges are merited, and such are within the scope of this invention.

For avoidance of doubt the term "treatment" includes therapeutic and/or prophylactic treatment.

### Biological evaluation

### Platelet Aggregation

Healthy male Lewis rats (Iffa Credo, Lyon, France) received a single oral dose of Compound (A) (10 mg/kg body weight). Fresh venous blood from two rats was sampled before, and at 2, 10, and 24 h after Compound (A) administration, and centrifuged at 250 g for 3 min at ambient temperature. Platelet-rich plasma was prepared as previously described (Eckly et al., 2001). A 450-µl platelet suspension was stirred at 1100 rpm and activated by the addition of 5 µM ADP (Sigma, Saint Quentin-Fallavier, France) in a final volume of 500 µl. Aggregation was measured at ambient temperature by a turbidimetric method in a dual-channel aggregometer (Chrono-Log, West Havertown, PA, USA). The extent of aggregation was estimated by measuring the maximum curve height above baseline.

### AAA Model

Experimental aortic aneurysms were induced by implanting a segment of sodium dodecyl sulfate-decellularized guinea-pig aorta (xenogenic matrix) in rat aortas (Allaire E. et al. (1994), J Vasc Surg 19:446-456). This AAA model is characterized by immuno-inflammatory injury of the xenogeneic extracellular matrix (Allaire E et al. (1997), Surgery 122:73-81; Allaire E et al. (1996), Transplantation 62:794-803). Briefly, 46 male Lewis rats (250 g) and 46 guinea-pigs (280 g) (Iffa Credo, Lyon, France) were housed according to the principles of Laboratory Animal Care formulated by the European Union. Animals were anaesthetized by intraperitoneal injection of pentobarbital (5 mg/100 g body weight). Guinea-pig infrarenal aortas (1.5 cm) were sampled and decellularized by sodium dodecyl sulfate treatment to obtain tubes of intact extracellular matrix, which were orthotopically transplanted into the aorta of Lewis rats with 10/0 nylon interrupted sutures (xenograft) (Allaire et al., 1997; Allaire et al., 1996). The initial diameter of the graft was measured *in situ,* immediately after unclamping, under a binocular microscope. After 24 h, rats were randomly assigned to two equal groups, treated with Compound (A) (10 mg/kg body weight twice daily by oral administration) or with the diluent of Compound (A) (1% carboxymethylcellulose/0.1 % Tween-80 in water) for 10 days (D 10, *n* = 9 for each group) or 6 weeks (D42, *n* = 14 for each group). At sacrifice, rats were deeply anesthetized, the graft diameter was measured again, and the aorta was dissected out and fixed in 4% paraformaldehyde. Aortic samples were embedded in paraffin and cut at 5 µm.

### Histological and Immunohistochemical Analysis

Sections were stained with picro Sirius red to visualize collagen and fibrin and with orcein to visualize elastin. Immunohistochemistry was performed using anti-human α-actin (dilution 1:500, Dako, Trappes, France), anti-CD68 (macrophages, dilution 1:1000, Serotec, Cergy Saint-Christophe, France), anti-rat polymorphonuclear leukocytes (dilution 1:3000, Cedarlane, Ontario, Canada), anti-CD41 (integrin α_{IIb}, platelets, dilution 1:50, Santa Cruz Biotechnologies) and anti-MMP-9 (dilution 1:50, Calbiochem, Darmstadt, Germany) as primary antibodies and a peroxidase Vector ABC kit for detection (Vector Labs, Burlingame, CA, USA). Cells were counted with a grid in the microscope eyepiece and averaged in 4 symmetrical fields on the same slide. The size of the areas represented by thrombus, elastic fiber, AAA wall, and MMP-9- and CD41-positive staining were measured by morphometric analysis using Histolab software (Microvision Instruments, Evly, France). Thrombus areas were determined on Sirius red slides. The area occupied by positive staining was quantified by the color detecting mode of the computer program in the inner thrombus and outer wall.

### Statistical Analysis

Results are expressed as means ± S.D. The percentage of diameter increase was calculated as follows: (diameter at harvest - diameter at grafting) x 100 / diameter at grafting. Comparisons between the two groups were made using the nonparametric Mann-Whitney U test (Statview, version 4.5). The frequency of aneurysms of large diameter in the 2 different groups was analyzed by *X*² test. Simple regression analysis was used to test for correlations between various parameters. *P* < 0.05 was considered significant.

### Results

### Aneurysmal Diameter

Decellularized extracellular matrix xenografts developed into aneurysms, as shown by the increase in the external aneurysmal diameter observed in untreated control rats 10 days (204.6 ± 71.2%, *n* = 9) and 42 days (359.1 ± 113.3%, *n* = 14) after grafting (Fig. 1). Compound (A) treatment for 10 days did not modify the increase in external aneurysmal diameter (197.3 ± 52.8%, *n* = 9), as compared to untreated rats; the mean external aneurysmal diameter was similar between the two groups (2.22 ± 0.56 mm vs. 2.22 ± 0.70 mm, in Compound (A)-treated and control rats, respectively) (Fig. 1a,c). However, after 42 days of treatment, the mean external aneurysmal diameter was significantly lower in the Compound (A)-treated group (3.61 ± 1.46 mm, *n =* 14 vs. 5.21 +/- 1.22 mm, *n* = 14, in Compound (A)-treated and control rats, respectively), and the percent increase in external diameter was significantly reduced (Fig. 1b,c). Compound (A)-treatment thus prevented aneurysm expansion, as shown by the difference in the percent increase in aortic diameter observed in control rats between Days 10 and 42, which was reduced in the Compound (A) group (Fig. 1c). In addition, the incidence of larger aneurysms at Day 42 (diameter > 4 mm) predominated in the control group, as compared to the Compound (A)-treated group (*X²=11.6,P<0.001*).

### Mural Thrombus Formation

Experimental aneurysms that develop from decellularized extracellular matrix xenografts are characterized by the presence of a mural thrombus, as previously described (Touat et al., 2006) (Fig. 2a). In the control group, the rates of thrombus area/wall area represented 0.22 ± 0.14 at Day 10 and increased to 0.78 ± 0.89 by Day 42 (Fig. 2b). Treatment with Compound (A) significantly reduced the thrombus/wall area, both at Days 10 and 42 (0.07 ± 0.05 and 0.35 ± 0.44, respectively) (Fig. 2b). This relative reduction in thrombus area was associated with a significant decrease in the CD41-positive area (integrin α_{IIb}, a platelet marker) within the thrombus at 42 days (0.13 ± 0.16 and 0.68 ± 1.36 for Compound (A)-treated and control group rats, respectively, *P* = 0.005) (Fig. 2c). Lastly, there was a significant correlation between the thrombus area and the aneurysm diameter at Day 42 when all rats were considered (*R²* = 0.25, *P* = 0.0003). Intragroup analysis showed that Compound (A) treatment significantly reduced the slope(s) of the correlation as compared to the control group (Compound (A): *s* = 0.17, *R²* = 0.40; control: *s* = 0.30, *R²* = 0.13) (Fig. 2c), providing evidence for that inhibition of platelet activation by Compound (A) will limit experimental AAA progression.

### Inflammatory Infiltration

Aneurysm development is associated with an inflammatory process that involves polymorphonuclear leukocytes (PMN) and macrophages. Both inflammatory cells were present within the mural thrombus and the aneurysmal wall (Fig. 3). However, PMNs were mostly found within the mural thrombus where they represented the main inflammatory cell type. In contrast, macrophages predominated within the wall. Whereas PMN density decreased between Days 10 and 42, macrophage density increased (Fig. 3). Compound (A) treatment significantly inhibited the infiltration of PMNs and macrophages into the mural thrombus at Days 10 and 42 (Fig. 3). There was no change in macrophage infiltration into the aneurysmal wall.

### MMP-9 Expression in Aneurysm and Preserved Elastic Fibers in the Media

The expression of MMP-9, revealed by immunostaining, was detected in control rats both in the thrombus and in the aneurysmal wall (Fig. 4a). The MMP-9-positive area was greater within the thrombus than in the wall at Day 42. Compound (A)-treatment markedly reduced the MMP-9-positive area, both in the thrombus and the wall (Fig. 4a). In addition, there was a significant positive correlation between thrombus area and MMP-9 expression within the thrombus at Day 42 when all rats were considered (*R²* = 0.79, *P* < 0.0001) (Fig. 4b). Reflecting the decreased MMP-9 expression, significant preservation of the elastic fibers of the media was observed with Compound (A) treatment at both timepoints (Fig. 5a). Although the relative area occupied by the elastic fibers remained constant in control rats between Day 10 and Day 42, elastic fiber area strongly increased in Compound (A)-treated rats (Fig. 5a).

### VSMC Colonization of the Mural Thrombus

SMCs colonized the decellularized graft and the part of the mural thrombus that developed close to the wall, as revealed by the α-actin positive staining within the wall and the thrombus (Fig. 5b). Moreover, the density of SMCs increased from Day 10 to Day 42, both within the wall and the thrombus, providing evidence of the tendency of the model to initiate a spontaneous healing process with time. Whereas Compound (A)-treatment for 10 days did not modify the density of SMCs within the thrombus compared to controls, treatment for 42 days significantly improved the SMC colonization of the thrombus (Fig. 5b). This was associated with a deposition of collagen, as shown by Sirius red staining (Fig. 2a). In contrast, treatment with Compound (A) did not affect SMC colonization of the wall after either 10 or 42 days (Fig. 5b).

### Conclusion

Prevention of AAA mural thrombus formation via inhibition of platelet activation by Compound (A) limited experimental AAA progression. By blocking the platelet activation and reducing the mural thrombus formation, Compound (A) decreased the recruitment of leukocytes in the thrombus and its subsequent enrichment with proteases, leading to the preservation of wall integrity and to the initiation of healing as revealed by the enhancement of mesenchymatous cell colonization.

## Claims

1. Use of the compound {1*S*-[1α,2α,3β(1S*,2*R**),5β]}-3-(7-{[2-(3,4-difluorophenyl)cyclopropyl]amino}-5-(propylthio)-3*H*-1,2,3-triazolo[4,5-*d*]pyrimidin-3-yl)-5-(2-hydroxyethoxy)cyclopentane-1,2-diol for the manufacture of a medicament for the treatment of abdominal aortic aneurysms.

2. The compound {1*S*-[1α,2α,3β(1*S**,2*R**),5β]}-3-(7-{[2-(3,4-difluorophenyl)cyclopropyl]amino}-5-(propylthio)-3*H*-1,2,3-triazolo[4,5-*d*]pyrimidin-3-yl)-5-(2-hydroxyethoxy)cyclopentane-1,2-diol for use in the treatment of abdominal aortic aneurysms.

## Patentansprüche

1. Verwendung der Verbindung {1S-[1α,2α,3β(1*S**,2*R**),5β]}-3-(7-{[2-(3,4-Difluorphenyl)cyclopropyl]amino}-5-(propylthio)-3*H-*1,2,3-triazolo[4,5-*d*]pyrimidin-3-yl)-5-(2-hydroxyethoxy)cyclopentan-1,2-diol zur Herstellung eines Medikaments zur Behandlung von Bauchaortenaneurysmen.

2. Die Verbindung {1S-[1α,2α,3β(1*S**,2*R**),5β]}-3-(7-{[2-(3,4-Difluorphenyl)cyclopropyl]amino}-5-(propylthio)-3*H*-1,2,3-triazolo[4,5-*d*]pyrimidin-3-yl)-5-(2-hydroxyethoxy)cyclopentan-1,2-diol zur Verwendung bei der Behandlung von Bauchaortenaneurysmen.

## Revendications

1. Emploi du composé {1*S*-[1α,2α,3β (1*S**,2*R**),5β]}-3-(7-{[2-(3,4-difluorophényl)cyclopropyl]amino}-5-(propylthio)-3*H-*1,2,3-triazolo[4,5-*d*]pyrimidin-3- yl)-5-(2-hydroxyéthoxy)cyclopentane-1,2-diol dans la fabrication d'un médicament destiné au traitement des anévrismes de l'aorte abdominale.

2. Composé {1*S*-[1α,2α,3β(1*S**,2*R**),5β]}-3-(7-{[2-(3,4-difluorophényl)cyclopropyl]amino}-5-(propylthio)-3*H*-1,2,3-triazolo[4,5-*d*]pyrimidin-3-yl)-5-(2-hydroxyéthoxy)cyclopentane-1,2-diol pour emploi dans le traitement des anévrismes de l'aorte abdominale.
